# EUROPÄISCHE PATENTSCHRIFT

(11) **EP 2 408 606 B1**
(45) Veröffentlichungstag und Bekanntmachung des Hinweises auf die Patenterteilung: **17.04.2013**
(21) Anmeldenummer: 10713816.6
(22) Anmeldetag: 15.03.2010
(51) Int. Cl.: B29C 49/20, A61F 7/08, B65D 41/04

(54) **WÄRMFLASCHE**
HOT-WATER BOTTLE
BOUILLOTTE

(30) Priorität: 17.03.2009 DE 102009013231
(43) Veröffentlichungstag der Anmeldung: 25.01.2012
(73) Patentinhaber: Frosch, Hugo, 86470 Thannhausen (DE)
(72) Erfinder: Frosch, Hugo, 86470 Thannhausen (DE)
(74) Vertreter: Binder, Armin
(86) Internationale Anmeldenummer: PCT/EP2010/001628
(87) Internationale Veröffentlichungsnummer: WO 2010/105792

(56) Entgegenhaltungen:
- EP-A1- 1 666 370
- WO-A1-2007/147206
- DE-A1- 19 730 800
- DE-B- 1 033 850
- JP-A- 7 125 055

## Beschreibung

Die Erfindung betrifft eine Warmflasche, aufweisend einen Hohlkörper, der mit einer Flüssigkeit befüllt werden kann, einen mit dem Hohlkörper verbundenen Einfüllstutzen zum Befüllen des Hohlkörpers, und einen Deckel zum Verschließen des Einfüllstutzens wobei die Wärmflasche durch ein Blasformverfahren hergestellt ist.

Wärmflaschen sind allgemein bekannt und sowohl zur wärme- als auch zur kältetherapeutischen Behandlung von Personen verwendbar. Im Allgemeinen werden Wärmflaschen aus einem elastischen Material hergestellt, so dass sich die Wärmflasche bedingt durch das Eigengewicht der Wasserfüllung an die Körperform der zu behandelnden Person anschmiegt. In der Gebrauchsmusterschrift DE 84 10 981 wird eine derartige Wärmflasche beschrieben. Zum Befüllen der Wärmflasche besitzt diese eine Einfüllöffnung, die mit Hilfe eines in der Öffnung verklemmten Gewindeeinlegeteils mit Innengewinde und eines Deckels mit einem Außengewinde zugeschraubt werden kann. Das Gewindeeinlegeteil besteht hier aus einem Mantelrohr mit angeformtem Schraubgewinde und Flanschkragen. Diese Bauteile sind aus einem wesentlich härteren Material - meist aus Metall oder einem harten Kunststoff - gefertigt als der elastische Körper der Wärmflasche, um eine mechanisch feste Verbindung und damit einen wasserdichten Verschluss zu gewährleisten.

Zusätzlich ist es aus der Patentanmeldung DE 197 30 800 A1 bekannt, Wärmflaschen aus einem thermoplastischen Material mittels einer Blastechnik herzustellen. In dieser Schrift wird ein Verfahren zur Herstellung einer mit einem Innengewindeeinsatz aus einem harten Kunststoffmaterial versehenen Wärmflasche aus einem weichelastischen, thermoplastischen Material beschrieben. Dazu wird ein extrudierter, aufzublasender Schlauch des thermoplastischen Materials zwischen zwei geöffneten Werkzeughälften gebracht. Für das Quetschen des Schlauchmaterials in die Bohrungen, die radiale Ringnut und die Ausbildung der Dichtlippe ist es notwendig, an dieser Stelle den Schlauch zu verdicken, um genügend zusätzliches Material zur Verfügung zu haben. Der Innengewindeeinsatz wird mit Hilfe eines Blasdorns in den Schlauch eingebracht. Zum Umquetschen dieses Gewindeeinsatzes wird der Blasdorn feststehend unterhalb der Form als so genannte Unterkalibrierung angebracht. Dadurch ist die Wärmflaschenform mit dem Trichter und dem Gewindeeinlegeteil nach unten gerichtet, so dass die Wärmflasche über Kopf steht. Als Folge dessen befindet sich die Dickstelle am Anfang des extrudierten Schlauches und darf somit eine bestimmte Länge, typischerweise circa fünf Zentimeter, nicht überschreiten.

Da das thermoplastische Schlauchmaterial nach dem Austritt aus der Düse eine Temperatur oberhalb der Erweichungstemperatur aufweist, zieht sich der extrudierte Schlauch durch sein Eigengewicht zusätzlich in die Länge. Diese Dehnung ist in jedem Fertigungszyklus unterschiedlich und kann nicht exakt reproduziert werden. Dies führt zu einer unterschiedlichen vertikalen Positionierung der Dickstelle innerhalb der Wärmflasche, also mal oberhalb des Gewindes und mal unterhalb an der Schulter der Wärmflasche.

Verstärkt wird dieser Effekt der unterschiedlichen Schlauchdehnung noch dadurch, dass die Dickstelle sich an der entferntesten Stelle von der Düse befindet, an der sich die Dehnung sowieso am stärksten auswirkt. Wird die Dickstelle zum Trichter hin verschoben und verpresst, also kalibriert, fehlt das Material zum Auffüllen der Ringnut und damit auch der Bohrungen. Dies führt dazu, dass das Gewindeeinlegeteil nicht ausreichend verankert wird.

Ist die Dickstelle mehr zur Schulter hin verschoben, fehlt Material zur Bildung der Dichtlippe, so dass diese nicht vollständig ausgebildet werden kann, und damit die Flasche durch den Verschlussdeckel nicht abgedichtet werden kann und damit unbrauchbar ist. Ein weiterer Nachteil, wenn die Dickstelle zu weit in den Schulterbereich hineinragt, ist, dass diese dadurch in den Quetschrand der Wärmflasche hineinkommt. Dadurch ist die Ausbildung des Quetschrandes ungünstig und sie kann im Gebrauch aufreißen. Die Wärmflasche kann dadurch sogar unbrauchbar sein.

Nachteilig an dem beschriebenen Verfahren ist der große Produktionsaufwand durch das Bereitstellen zusätzlicher Aggregate für das Sortieren und Zuführen des Innengewindeeinsatzes und die zusätzlichen Materialkosten, da der Innengewindeeinsatz aus einem anderen, härteren Material gefertigt wird als der eigentliche Körper der Wärmflasche. Zusätzlich ist aufgrund der beschriebenen Mängel, wie fehlerhafter Verschluss der Wärmflasche und aufreißender Quetschrand, eine kontinuierliche Serienfertigung ohne erheblichen Kontrollaufwand nicht möglich, wenn nicht sogar unmöglich, weil zum Beispiel der fehlerhafte Quetschrand, der sich durch die Dickstelle ergibt, von außen nicht sichtbar ist.

Des Weiteren sind auch Wärmflaschen aus Metall bekannt. In der Patentschrift DE 575 791 A wird eine metallene Wärmflasche mit einem wasserdichten Verschluss beschrieben. Die Wärmflasche weist hier einen nach außen ragenden Kopf auf, der mit einem Außengewinde versehen ist, so dass eine mit einem Innengewinde versehene Verschlusshaube aufgeschraubt werden kann und ein wasserdichter Verschluss möglich ist.

Es sind bisher also entweder Wärmflaschen aus einem thermoplastischen Material mit einem zusätzlichen Gewindeeinlegeteil mit Innengewinde oder Metall-Wärmflaschen mit Außengewinde bekannt.

Bei den bisher bekannten Wärmflaschen aus Thermoplasten benötigt man folglich zum Verschließen der Einfüllöffnung ein zusätzlich in die Öffnung einzusetzendes starres Gewindeeinlegeteil, in welches der Deckel eingeschraubt werden kann. Das Anfertigen und Einbringen des Gewindeeinlegeteils ist kostenintensiv und aufwendig. Ohne das zusätzliche Gewindeeinlegeteil hat das elastische Wärmflaschenmaterial aber nicht die nötige Härte und die funktionellen Eigenschaften, um einen formschlüssigen, wasserdichten Verschluss mit einem eingeschraubten Deckel zu ermöglichen.

Die ebenfalls bekannten Metall-Wärmflaschen mit einem Außengewinde sind in ihrer Anwendung unkomfortabel, da sich die starre Metallform nicht den persönlichen Körperformen anpasst.

Aus der JP 7 125055 A ist noch eine mittels eines Blasformverfahrens hergestellte Trinkflasche bekannt. Die Trinkflasche weist einen Hohlkörper und einen Einfüllstutzen mit einem Außengewinde auf. Hierbei sind der Einfüllstutzen und das Außengewinde aus mehreren Schichten aus unterschiedlichen Materialien gefertigt.

Es ist daher Aufgabe der Erfindung eine neuartige Wärmflasche zu beschreiben, die zum Einen durch ein einfaches Herstellungsverfahren mit niedrigen Stückkosten produziert werden kann und zum Anderen den bestmöglichen Komfort in der Anwendung bietet.

Diese Aufgabe wird durch die Merkmale des unabhängigen Patentanspruches gelöst. Vorteilhafte Weiterbildungen der Erfindung sind Gegenstand untergeordneter Ansprüche.

Der Erfinder hat erkannt, dass es möglich ist, eine Wärmflasche aus einem thermoplastischen Material herzustellen und diese ohne zusätzliche Gewindeteile wasserdicht zu verschließen. Durch das so genannte Blasformverfahren ist es möglich, den Verschlussbereich, das heißt den Einfüllstutzen mit Gewinde, und den Körper der Wärmflasche einstückig aus dem selben weichen thermoplastischen Material zu fertigen. Beim Ausblasen des Rohmaterials in die spätere Form der Wärmflasche kann man eine gleichmäßige Wandstärke des Hohlkörpers sowie des Einfüllstutzens der Wärmflasche erreichen. Somit ist der Einfüllstutzen stabil genug, dass das an ihm angeformte Außengewinde in Verbindung mit einem Innengewinde im Inneren eines aufgeschraubten Deckels einen mechanisch festen Verschluss gewährleisten kann.

Bei diesem Herstellungsverfahren fallen also zusätzliche Materialkosten für ein separates Gewinde und dessen aufwendiges Einsetzen in den Einfüllstutzen der Wärmflasche weg, wodurch sich das Verfahren gegenüber dem beschriebenen Verfahren des Patentes DE 197 30 800 A1 unterscheidet. Des Weiteren bleibt der Komfort in der Anwendung durch das thermoplastische Material der Wärmflasche, das sich den Körperformen anpassen kann, erhalten. Weiterhin ist die umweltbelastende Entsorgung des bisher verwendeten PVC-Materials nicht mehr zwangsläufig nötig, da es möglich ist, ein anderes Material zu verwenden.

Entsprechend diesem Grundgedanken schlägt der Erfinder vor, eine Wärmflasche, aufweisend einen Hohlkörper, der mit einer Flüssigkeit befüllt werden kann, einen mit dem Hohlkörper verbundenen Einfüllstutzen zum Befüllen des Hohlkörpers, und einen Deckel zum Verschließen des Einfüllstutzens, wobei die Wärmflasche durch ein Blasformverfahren hergestellt ist, dahingehend zu verbessern, dass der Einfüllstutzen ein Außengewinde aufweist, der Deckel ein Innengewinde zum Aufschrauben auf den Einfüllstutzen aufweist, und der Einfüllstutzen mit Außengewinde und der Hohlkörper einstückig aus demselben Material ausgeführt sind.

Vorteilhafterweise kann der einstückige Verbund aus Hohlkörper und Einfüllstutzen aus einem thermoplastischen Elastomer hergestellt werden. Diese Materialgruppe ist vor allem für die Verarbeitung mit einem Blasformverfahren geeignet und bietet durch seine Eigenschaften, beispielsweise hohe Flexibilität und Elastizität, eine hohe Qualität der Wärmflasche, in der Anwendung.

Des Weiteren ist es vorteilhaft, den Deckel aus einem Thermoplast zu fertigen. Hierbei ist es allerdings sinnvoll, ein Thermoplast zu verwenden, das härter ist als das Material des Hohlkörpers und des Einfüllstutzens. Es eignen sich zum Beispiel die Materialien Polyamid, Polyethylen oder Polypropylen. Bei gleicher Harte des Materials wäre die Reibung am Gewinde so groß, dass ein Aufschrauben des Deckels und damit ein wasserdichter Verschluss kaum möglich wäre.

Werden bei der Herstellung der Wärmflasche die Aspekte kostengünstige Produktion und umweltschonende Entsorgung vernachlässigt, kann die Wärmflasche auch aus PVC hergestellt werden. Dabei sollte allerdings darauf geachtet werden, dass, wenn Deckel und Hohlkörper mit Einfüllstutzen aus PVC hergestellt sind, der Weichmacher aus dem weicheren PVC des Hohlkörpers und des Einfüllstutzens in das härtere PVC des Deckels eindringen kann. Als Folge dessen weicht der Deckel auf bis die Härte der Bauteile ausgeglichen ist und somit der erfindungsgemäße Vorteil des wasserdichten Verschlusses nicht mehr gewährleistet ist.

Um einen wasserdichten Verschluss der erfindungsgemäßen Wärmflasche zu ermöglichen, kann in einer bevorzugten Ausführungsform der Deckel einen Stützzylinder aufweisen. Dieser Stützzylinder befindet sich gegenüber dem Innengewinde an der Deckelaußenseite und schließt bei aufgeschraubtem Deckel den Einfüllstutzen ein. Des Weiteren kann der Stützzylinder einen zum Innengewinde zeigenden, umlaufenden Keil aufweisen. Bei aufgeschraubtem Deckel presst sich dann der Keil gegen den Einfüllstutzen im Bereich des Stützzylinders und es wird somit eine erste Auslaufsperre für die Flüssigkeit bei befüllter Wärmflasche verwirklicht.

Zusätzlich kann zwischen dem Stützzylinder und dem Innengewinde eine Wulst ausgebildet sein, die im aufgeschraubten Zustand des Deckels gegen den stirnseitigen Bereich des Einfüllstutzens drückt. Dabei wird das härtere Material des Deckels in das weichere Material des Einfüllstutzens gedrückt, so dass eine zweite Abdichtung entsteht. Mit diesen beiden Abdichtungen ist ein vollständig wasserdichter Verschluss des Hohlkörpers gewährleistet.

Vorteilhafterweise kann beim Blasformen der Wärmflasche im Bereich des Einfüllstutzens ein Überquetschen stattfinden. Dabei kann die Größe des Schlauchrohlings so gewählt werden, dass nahezu keine bis nur eine minimale Dehnung des Schlauches bei der Formung in die eigentliche Form der Wärmflasche entsteht. Dadurch kann das Überquetschen vor allem an den Seitenbereichen des Hohlkörpers vermieden werden, so dass hier kein Quetschrand entsteht. Ein Vermeiden des Quetschrandes wirkt sich vorteilhaft auf die Haltbarkeit und Stabilität der Wärmflasche aus. Dagegen können aus dem überquetschten Material seitlich des Einfüllstutzens, ohne Einbuße in der Stabilität zu bewirken, zum Beispiel die Haltelaschen geformt werden.

Des Weiteren kann der Hohlkörper mit mindestens einer Haltelasche versehen werden, die vorteilhafterweise an mindestens einer Seite ein Profil aufweist, welches das Greifen der Haltelasche erleichtert. In einer Ausführungsform kann die mindestens eine Haltelasche durch eine Vergrößerung des Quetschrandes geformt werden. Die Haltelaschen können vielfältig verwendet werden, beispielsweise zum Fixieren der Wärmflasche an einem bestimmten Körperteil oder zum Aufhängen der Wärmflasche beim Ablassen der Flüssigkeit. Zusätzlich kann durch die, an einer vorteilhaften Position angebrachten, Haltelaschen das Risiko einer Verbrennung des Benutzers beim Befüllen der Wärmflasche mit heißer Flüssigkeit gesenkt werden, da der Benutzer die Wärmflasche nicht unbedingt am Einfüllstutzen festhalten muss, sondern die Haltelasche verwenden kann.

Es wird darauf hingewiesen, dass die erfindungsgemäße Wärmflasche auch für die Anwendung mit einer kalten Flüssigkeit geeignet ist.

Weiterhin kann es vorteilhaft sein, wenn die Oberfläche des Deckels nicht glatt ausgestaltet ist, sondern ein Profil aufweist, zum Beispiel Griffrillen. Das Profil ermöglicht dann ein besseres Greifen und festeres Zuschrauben des Deckels auf die Wärmflasche.

Im Folgenden wird die Erfindung anhand der bevorzugten Beispiele mit Hilfe der Figuren näher beschrieben, wobei darauf hingewiesen wird, dass nur die für das unmittelbare Verständnis der Erfindung wesentlichen Elemente gezeigt sind.

Es zeigen im Einzelnen:
- FIG 1:: Vorderansicht einer erfindungsgemäßen Wärmflasche mit Deckel;
- FIG 2:: Seitenansicht einer erfindungsgemäßen Wärmflasche mit Deckel;
- FIG 3:: Seitenansicht einer erfindungsgemäßen Wärmflasche mit aufgeschraubtem Deckel;
- FIG 4:: Draufsicht einer erfindungsgemäßen Wärmflasche mit aufgeschraubtem Deckel;
- FIG 5:: Halbprofil einer erfindungsgemäßen Wärmflasche;
- FIG 6:: Ausschnitt eines Querschnitts durch den Einfüllstutzen mit aufgeschraubtem Deckel.

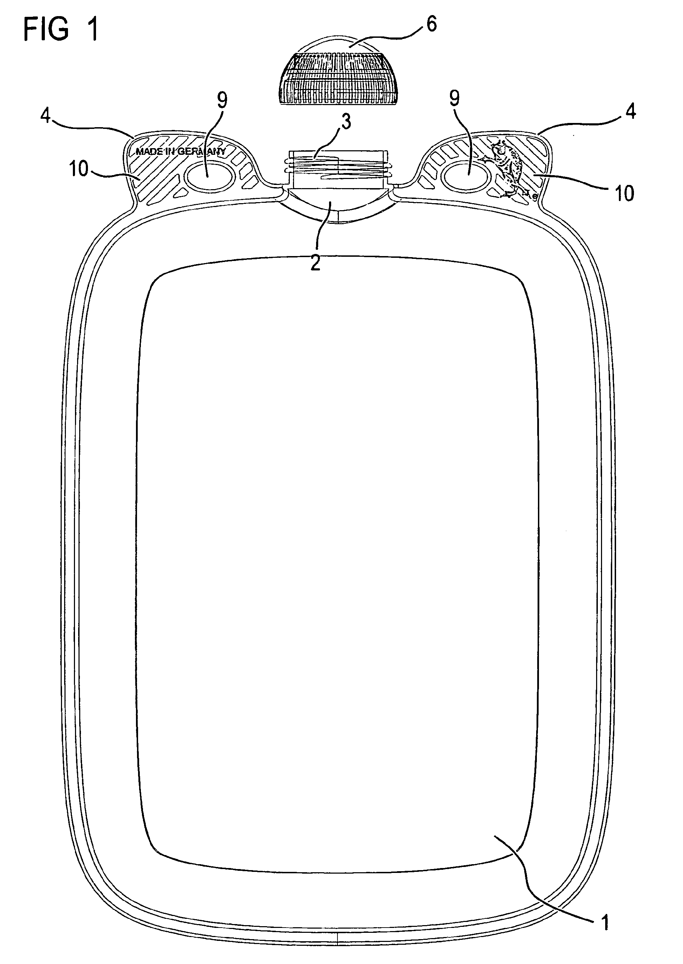

Die **Figur 1** zeigt eine Ausführungsform der erfindungsgemäßen Wärmflasche in Vorderansicht mit ebenfalls abgebildetem Deckel 6. Den größten Teil der Wärmflasche stellt ein näherungsweise rechteckiger Hohlkörper 1 dar. An ihm befindet sich ein Einfüllstutzen 2, durch den der Hohlkörper 1 mit einer heißen oder kalten Flüssigkeit befüllt werden kann. Der Hohlkörper 1 und der Einfüllstutzen 2 sind einstückig aus demselben thermoplastischen Elastomer mittels eines Blasformverfahrens gefertigt. Um den Einfüllstutzen 2 verschließen zu können, weist dieser ein Außengewinde 3 auf, auf welches der Deckel 6 aufgeschraubt werden kann.

In dieser Ausführungsform befinden sich seitlich des Einfüllstutzens 2 zwei Haltelaschen 4, die jeweils ein Griffloch 9 aufweisen. An den Haltelaschen 4 kann die Wärmflasche zum Beispiel während des Füllvorgangs fixiert werden, so dass die Verbrennungsgefahr minimiert wird. Auf der hier gezeigten Vorderseite der Wärmflasche, besitzen die Haltelaschen 4 ein Profil 10, bestehend aus länglichen Griffrillen. Dieses Profil 10 verbessert nochmals die Handhabung der Wärmflasche.

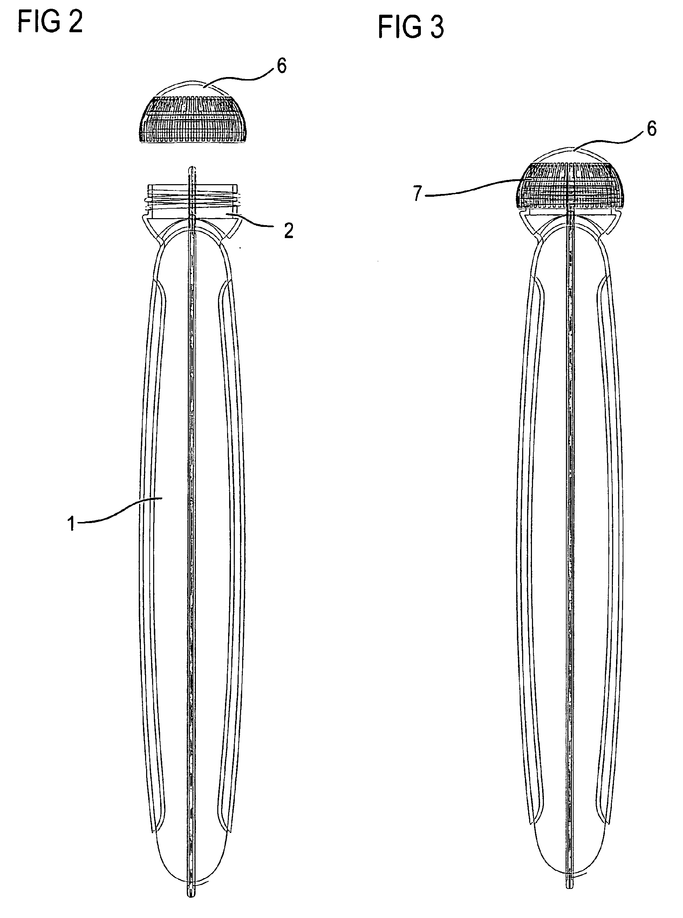

In der **Figur 2** ist eine Seitenansicht der erfindungsgemäßen Wärmflasche aus der Figur 1 mit Deckel 6 abgebildet. In dieser Darstellung ist deutlich zu erkennen, dass der Hohlkörper 1 einen sich zum Einfüllstutzen 2 hin verjüngenden Querschnitt aufweist, wobei sich der Einfüllstutzen 2 trichterförmig zu seiner Öffnung hin vergrößert. Die größere Öffnung des Einfüllstutzens 2 kann so das Einfüllen von Flüssigkeit erleichtern.

Die **Figur 3** zeigt ebenfalls die erfindungsgemäße Wärmflasche aus der Figur 2. In dieser Darstellung ist der Einfüllstutzen jedoch durch den aufgeschraubten, halbkugelförmigen Deckel 6 verschlossen. Dieser weist an seiner Außenseite ein Profil 7 auf, welches ein besseres Zugreifen und damit ein festeres Zuschrauben ermöglicht. Um einen festen Verschluss der Wärmflasche zu gewährleisten, sollte der Deckel 6 aus einem härteren Material gefertigt werden als der Hohlkörper und der Einfüllstutzen. Dazu eignen sich zum Beispiel die Thermoplaste Polyamid, Polyethylen oder Polypropylen.

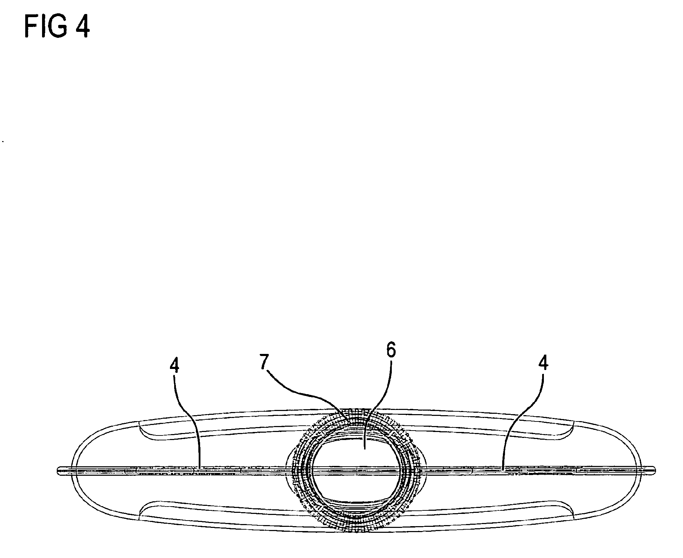

Die **Figur 4** zeigt die erfindungsgemäße Wärmflasche aus Figur 1 in Draufsicht mit aufgeschraubtem Deckel 6. Zu sehen ist der Deckel 6 mit dem Profil 7 und den zwei Haltelaschen 4 links und rechts des Einfüllstutzens.

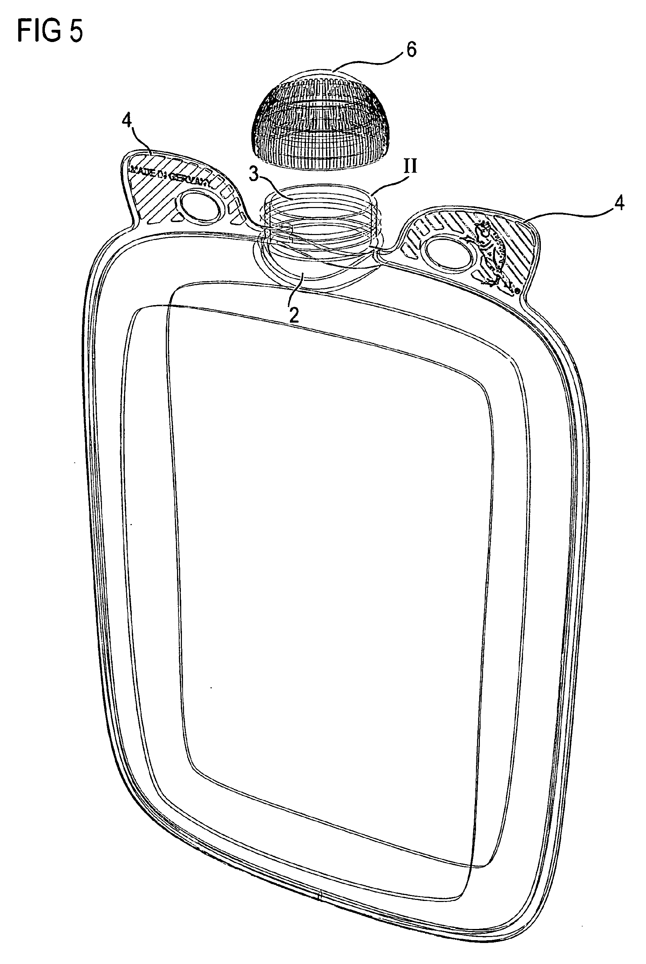

Die **Figur 5** zeigt ein Halbprofil der erfindungsgemäßen Wärmflasche aus der Figur 1. Hier ist vor allem der Einfüllstutzen 2 mit dem Außengewinde 3 und der Dichtfläche II gut zu erkennen. Im Inneren des Deckels 6 befindet sich ein hier nicht sichtbares Innengewinde, welches durch Zusammenwirken mit dem Außengewinde 3 des Einfüllstutzens 2 den Einfüllstutzen 2 und Deckel 6 ineinander presst und so einen wasserdichten Verschluss des Einfüllstutzens 2 ermöglicht.

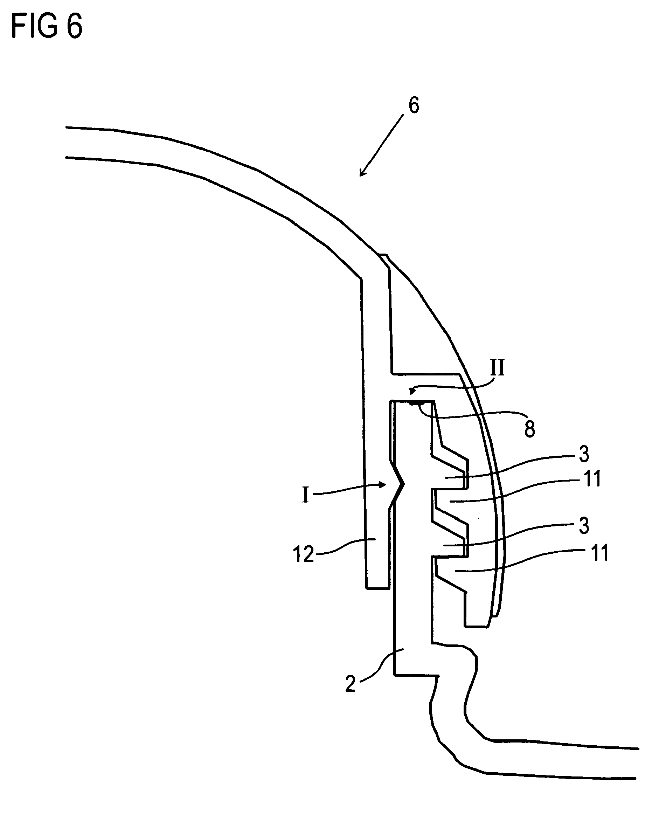

Die **Figur 6** zeigt einen Ausschnitt aus einem Querschnitt durch den Einfüllstutzen 2 mit aufgeschraubtem Deckel 6. In diesem Querschnitt ist besonders das Innengewinde 11 des Deckels 6 und das Außengewinde 3 des Einfüllstutzens 2 gut zu erkennen. Zusätzlich weist in dieser Ausführung der Deckel 6 einen Stützzylinder 12 auf. Der Stützzylinder 12 ist gegenüber dem Innengewinde 11 angeordnet, so dass der Einfüllstutzen 2 bei aufgeschraubtem Deckel 6 auf seiner Außenseite vom Innengewinde 11 des Deckels 6 und auf seiner Innenseite vom Stützzylinder 12 eingeschlossen ist. Dabei ist der Stützzylinder 12 so gestaltet, dass er einen umlaufenden Keil aufweist. Dieser Keil wird beim Aufschrauben des Deckels 6 an den Einfüllstutzen 2 gepresst. Dadurch bilden der weiche Einfüllstutzen und der härtere Stützzylinder 12 eine erste Abdichtung I aus.

Zusätzlich befindet sich zwischen dem Innengewinde 11 und dem Stützzylinder 12 eine Wulst 8. Diese Wulst 8 wird, wenn der Deckel 6 fest aufgeschraubt ist, an das obere Ende des Einfüllstutzens 2 gepresst. Dabei drückt sich das härtere Deckelmaterial in das weichere Material des Einfüllstutzens 2 und es entsteht eine weitere Abdichtung II.

Insgesamt wird mit der Erfindung also eine Wärmflasche, aufweisend einen Hohlkörper, der mit einer Flüssigkeit befüllt werden kann, einen mit dem Hohlkörper verbundenen Einfüllstutzen zum Befüllen des Hohlkörpers, und einen Deckel zum Verschließen des Einfüllstutzens, vorgeschlagen, die derart weiterverbessert wurde, dass die Wärmflasche durch ein Blasformverfahren hergestellt ist, wobei der Einfüllstutzen und der Hohlkörper einstückig ausgeführt sind, der Einfüllstutzen ein Außengewinde, und der Deckel ein Innengewinde aufweist.

Durch dieses Herstellungsverfahren ist es möglich, den Anwendungskomfort einer herkömmlichen Wärmflasche aus einem thermoplastischen Material mit der einfachen Herstellungsart einer herkömmlichen Metall-Wärmflasche zu vereinen.

Es versteht' sich, dass die vorstehend genannten Merkmale der Erfindung nicht nur in der jeweils angegebenen Kombination, sondern auch in anderen Kombinationen oder in Alleinstellung verwendbar sind, ohne den Rahmen der Erfindung zu verlassen.

### Bezugszeichenliste

- 1: Hohlkörper
- 2: Einfüllstutzen
- 3: Außengewinde
- 4: Haltelasche
- 6: Deckel
- 7: Profil am Deckel
- 8: Wulst
- 9: Griffloch
- 10: Profil an der Haltelasche
- 11: Innengewinde
- 12: Stützzylinder

- I: erste Abdichtung
- II: zweite Abdichtung

## Patentansprüche

1. Wärmflasche, aufweisend:
einen Hohlkörper (1), der mit einer Flüssigkeit befüllt werden kann,
einen mit dem Hohlkörper (1) verbundenen Einfüllstutzen (2) zum Befüllen des Hohlkörpers (1), und
einen Deckel (6) zum Verschließen des Einfüllstutzens (2), wobei
die Wärmflasche durch ein Blasformverfahren hergestellt ist,
**dadurch gekennzeichnet, dass**
der Einfüllstutzen (2) ein Außengewinde (3) aufweist, der Deckel (6) ein Innengewinde zum Aufschrauben auf den Einfüllstutzen (2) aufweist, und
der Einfüllstutzen (2) mit Außengewinde (3) und der Hohlkörper (1) einstückig aus demselben Material ausgeführt sind.

2. Wärmflasche gemäß dem voranstehenden Patentanspruch 1, **dadurch gekennzeichnet, dass** der Hohlkörper (1) und der Einfüllstutzen (2) mit Außengewinde (3) aus einem thermoplastischen Elastomer bestehen.

3. Wärmflasche gemäß dem voranstehenden Patentanspruch 2, **dadurch gekennzeichnet, dass** der Deckel (6) aus einem Thermoplast besteht, welches härter als das für den Hohlkörper (1) und den Einfüllstutzen (2) verwendete thermoplastische Elastomer ist.

4. Wärmflasche gemäß dem voranstehenden Patentanspruch 3, **dadurch gekennzeichnet, dass** der Deckel (6) aus einem Thermoplast der nachfolgenden Liste besteht: Polyamid, Polyethylen und Polypropylen.

5. Wärmflasche gemäß einem der voranstehenden Patentansprüche 1 bis 4, **dadurch gekennzeichnet, dass** der Deckel (6) gegenüber dem Innengewinde (11) einen Stützzylinder (12) aufweist, der den Einfüllstutzen (2) einschließt.

6. Wärmflasche gemäß dem voranstehenden Patentanspruch 5, **dadurch gekennzeichnet, dass** der Stützzylinder (12) über teilweise Höhenbereiche keilförmig ausgebildet ist.

7. Wärmflasche gemäß einem der voranstehenden Patentansprüche 5 bis 6, **dadurch gekennzeichnet, dass** zwischen dem Stützzylinder (12) und dem Innengewinde (11) eine Wulst (8) ausgebildet ist, die im aufgeschraubten Zustand des Deckels (6) gegen den stirnseitigen Bereich des Einfüllstutzens (2) drückt.

8. Wärmflasche gemäß einem der voranstehenden Patentansprüche 1 bis 7, **dadurch gekennzeichnet, dass** bei dem Blasformverfahren im Bereich des Einfüllstutzens (2) ein Überquetschen stattfindet.

9. Wärmflasche gemäß einem der voranstehenden Patentansprüche 1 bis 8, **dadurch gekennzeichnet, dass** der Hohlkörper (1) mindestens eine Haltelasche (4) aufweist.

10. Wärmflasche gemäß dem voranstehenden Patentanspruch 9, **dadurch gekennzeichnet, dass** die mindestens eine Haltelasche (4) auf mindestens einer Seite ein Profil (10) aufweist.

11. Wärmflasche gemäß einem der voranstehenden Patentansprüchen 1 bis 10, **dadurch gekennzeichnet, dass** der Deckel (6) ein Profil (7) aufweist.

## Claims

1. Hot-water bottle, having:
a hollow body (1), which can be filled with a liquid,
a filler neck (2) connected to the hollow body (1) for filling the hollow body (1), and
a cap (6) for closing the filler neck (2), wherein
the hot-water bottle is produced by a blow moulding process,
**characterized in that**
the filler neck (2) has an external thread (3), the cap (6) has an internal thread for being screwed onto the filler neck (2), and
the filler neck (2) with the external thread (3) and the hollow body (1) are formed in one piece from the same material.

2. Hot-water bottle according to the preceding patent Claim 1, **characterized in that** the hollow body (1) and the filler neck (2) with the external thread (3) consist of a thermoplastic elastomer.

3. Hot-water bottle according to the preceding patent Claim 2, **characterized in that** the cap (6) consists of a thermoplastic which is harder than the thermoplastic elastomer used for the hollow body (1) and the filler neck (2).

4. Hot-water bottle according to the preceding patent Claim 3, **characterized in that** the cap (6) consists of a thermoplastic from the following list: polyamide, polyethylene and polypropylene.

5. Hot-water bottle according to one of the preceding patent Claims 1 to 4, **characterized in that** the cap (6) has, opposite the internal thread (11), a supporting cylinder (12), which encloses the filler neck (2).

6. Hot-water bottle according to the preceding patent Claim 5, **characterized in that** the supporting cylinder (12) is formed with a wedge shape over partial regions of the height.

7. Hot-water bottle according to one of the preceding patent Claims 5 to 6, **characterized in that** a bead (8) is formed between the supporting cylinder (12) and the internal thread (11), which bead presses against the region on the face of the filler neck (2) when the cap (6) is screwed on.

8. Hot-water bottle according to one of the preceding patent Claims 1 to 7, **characterized in that** over-pinching takes place in the region of the filler neck (2) during the blow moulding process.

9. Hot-water bottle according to one of the preceding patent Claims 1 to 8, **characterized in that** the hollow body (1) has at least one holding tab (4).

10. Hot-water bottle according to the preceding patent Claim 9, **characterized in that** the at least one holding tab (4) has a profile (10) on at least one side.

11. Hot-water bottle according to one of the preceding patent Claims 1 to 10, **characterized in that** the cap (6) has a profile (7).

## Revendications

1. Bouillotte, comportant:
- un corps creux (1), qui peut être rempli d'un liquide,
- un embout de remplissage (2) relié au corps creux (1) pour le remplissage du corps creux (1), et
- un couvercle (6) pour fermer l'embout de remplissage (2),
dans laquelle
- la bouillotte est fabriquée par un procédé de moulage par soufflage,
**caractérisée en ce que**
- l'embout de remplissage (2) présente un filet extérieur (3),
- le couvercle (6) présente un filet intérieur pour le visser sur l'embout de remplissage (2), et
- l'embout de remplissage (2) avec le filet extérieur (3) et le corps creux (1) sont réalisés en une seule pièce à partir du même matériau.

2. Bouillotte selon la revendication précédente 1, **caractérisée en ce que** le corps creux (1) et l'embout de remplissage (2) avec le filet extérieur (3) sont constitués d'un élastomère thermoplastique.

3. Bouillotte selon la revendication précédente 2, **caractérisée en ce que** le couvercle (6) est constitué d'un plastique thermodurcissable, qui est plus dur que l'élastomère thermoplastique utilisé pour le corps creux (1) et l'embout de remplissage (2).

4. Bouillotte selon la revendication précédente 3, **caractérisée en ce que** le couvercle (6) est constitué d'un plastique thermodurcissable de la liste suivante: polyamide, polyéthylène et polypropylène.

5. Bouillotte selon une des revendications précédentes 1 à 4, **caractérisée en ce que** le couvercle (6) présente, en face du filet intérieur (11), un cylindre de soutien (12) qui entoure l'embout de remplissage (2).

6. Bouillotte selon la revendication précédente 5, **caractérisée en ce que** le cylindre de soutien (12) est profilé en forme de coin sur des régions partielles en hauteur.

7. Bouillotte selon une des revendications précédentes 5 à 6, **caractérisée en ce que**, entre le cylindre de soutien (12) et le filet intérieur (11), est formé un rebord (8) qui, dans l'état vissé du couvercle (6), presse contre la région frontale de l'embout de remplissage (2).

8. Bouillotte selon une des revendications précédentes 1 à 7, **caractérisée en ce que**, lors du procédé de moulage par soufflage, il se produit un surserrage dans la région de l'embout de remplissage (2).

9. Bouillotte selon une des revendications précédentes 1 à 8, **caractérisée en ce que** le corps creux (1) présente au moins une patte de maintien (4).

10. Bouillotte selon la revendication précédente 9, **caractérisée en ce que** ladite au moins une patte de maintien (4) présente un profil (10) sur au moins un côté.

11. Bouillotte selon une des revendications précédentes 1 à 10, **caractérisée en ce que** le couvercle (6) présente un profil (7).
